# EUROPEAN PATENT SPECIFICATION

(11) **EP 0 926 256 B1**
(45) Date of publication and mention of the grant of the patent: **07.05.2003**
(21) Application number: 98905679.1
(22) Date of filing: 26.02.1998
(51) Int. Cl.: C23C 10/28, C23C 10/30, C23C 14/48, A61L 2/16

(54) **ANTIBACTERIAL METALLIC MATERIALS AND METHOD OF PRODUCTION THEREOF**
ANTIBAKTERIELLE METALLISCHE MATERIALIEN UND DEREN HERSTELLUNGSVERFAHREN
MATERIAUX METALLIQUES ANTIBACTERIENS ET PROCEDE DE PRODUCTION

(30) Priority: 26.02.1997 JP 4264497
(43) Date of publication of application: 30.06.1999
(73) Proprietor: SUMITOMO OSAKA CEMENT CO., LTD., Chiyoda-ku Tokyo 101 (JP)
(72) Inventor: SHIGERU, Keijiro;-Advanced Materials Div. Sumitomo, Funabbashi-shi;,Chiba 274-8601 (JP); INOUE, Yoshitomo;-Advanced Materials Div. Sumitomo, Funabbashi-shi;,Chiba 274-8601 (JP); AWANO, Yasuyuki;-Advanced Materials Div. Sumitomo, Funabaashi-shi;,Chiba 274-8601 (JP); YAZAWA, Takako;-Advanced Materials Div. Sumitomo, Funabashi-shi;,Chiba 274-8601 (JP)
(74) Representative: Werner, Dietrich H., Dr.-Ing.
(86) International application number: JP9800804
(87) International publication number: WO98038349

(56) References cited:
- WO-A-95/13704
- GB-A- 1 093 358
- JP-A- 56 081 666
- JP-B- 4 016 179
- DATABASE WPI Section Ch, Week 199635 Derwent Publications Ltd., London, GB; Class A32, AN 1996-350428 XP002136717 -& JP 08 165561 A (NIPPON LIGHT METAL CO), 25 June 1996 (1996-06-25)

## Description

### Technical Field

The present invention relates to a process for producing an antimicrobial metallic material, and particularly it relates to economical processes for producing metallic materials with excellent antimicrobial properties.

The term "antimicrobial" as used throughout this specification includes "antifungal" and "antialgal".

### Background Art

Conventional metallic materials have been used in a large variety of tools of the type which are required to be sanitary and hygienic. The reasons for this are believed to be not only their resistance against cleaning and disinfectant treatment, but also the fact that the metals themselves exhibit antimicrobial action known as the oligodynamic effect.

The oligodynamic effects of mercury (Hg), silver (Ag), copper (Cu), zinc (Zn), nickel (Ni), chromium (Cr), iron (Fe), molybdenum (Mo), etc. are well known, and among these mercury and silver have markedly superior oligodynamic effects.

However, mercury cannot be used as a metallic material because of its liquid state at room temperature, and while silver can be used as a metallic material, its high cost and susceptibility to darkening and softness have been problems which have limited its range of uses. Other metals have exhibited weaker antimicrobial properties than silver.

JP-A-56-81666 discloses a color-tinted hard watch case comprising an alloy of 30 to 50 % of Cr with 3 to 6 % of Al and the balance consisting of Ni and unavoidable impurities. The watch case is produced by age-hardening the alloy case, plating the alloy case with a noble metal (for example, gold, or silver, or platinum or its alloy to form a plating having a thickness of the 0.5 to 10 µm and then heat treating the plated watch case at a temperature identical to or lower than the age-hardening temperature to cause the plated metal to diffuse into the inside of the alloy watch case. The resultant plated and heat-treated watch case must be fully coated by the noble metal or alloy plating layer, and thus the surface of the alloy watch case, per se, cannot appear.

GB Patent No. 1,093,358 discloses a method for improving the corrosion resistance of high temperature base alloys. The method comprises depositing a layer of silver upon the surface of the alloy and then subjecting the coated alloy to a heat treatment to effect diffusion of at least major proportion of the silver into the alloy, depositing a layer of nickel upon the treated alloy surface, depositing a layer of aluminium upon the nickel and diffusing aluminium into the nickel layer to form a nickel-aluminium layer. The resultant coated alloy material must be coated by the Ni-Al layer, and thus the diffused silver cannot exhibit an anti-bacterial activity and the surface of the base alloy material cannot appear.

The present invention has been accomplished in the light of these problems of the prior art, and its concrete means for solving them is to provide antimicrobial metallic materials obtained by imparting silver metal-equivalent antimicrobial properties to non-silver metals which can be more economically produced, as well as processes for their production.

### Disclosure of the Invention

The antimicrobial metallic material produced by the process as described in claim 1 of the invention is characterized by having silver atoms diffused in a metallic material surface portion at a concentration of 0.5-10,000 ppm.

In an antimicrobial metallic material produced by the process of the invention, the silver atom-diffused portion in the metallic material surface portion preferably has a thickness of 30 µm or less.

In an antimicrobial metallic material produced by the process of the invention, the silver atoms are dispersed in the metallic material surface portion in such a manner that a portion of the silver, silver alloy or silver compound is exposed on the metallic material surface.

The production process for an antimicrobial metallic material of the invention is characterized by heat-diffusing silver atoms from a surface into the inside of a metallic material to form a silver atom-diffused surface layer; and removing an uppermost surface portion of the silver atom-diffused surface layer to cause a resultant surface of the metallic material on which the silver atoms are distributed in a density of 0.5 to 10,000 ppm, to be exposed.

### Brief Description of the Drawings

Fig. 1 is a cross-sectional diagram showing the state of distribution of silver atoms in an embodiment of an antimicrobial metallic material made according to the process of the invention.
Fig. 2 is a graph showing the silver atom concentration distribution in an embodiment of the stainless steel scissors described in Example 1.
Fig. 3 is a graph showing the silver atom concentration distribution in an embodiment of the steel blade described in Example 2.

### Best Mode for Carrying Out the Invention

Embodiments of the invention will now be explained in detail.

The embodiments described below, however, are intended to serve as concrete explanations for a better understanding of the invention, and unless specifically stated, do not limit the content of the invention.

### [Antimicrobial metallic material]

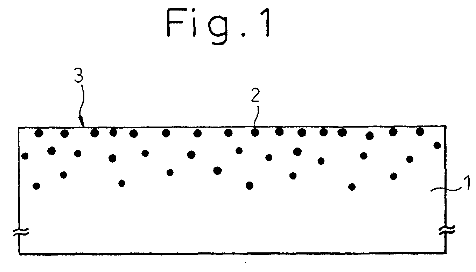

As shown in Fig. 1, a metallic material 1 with antimicrobial properties produced according to the process of the invention has silver atoms 2 diffused through its surface portion at a silver concentration of 0.5-10,000 ppm with the highest concentration on the surface 3 and decreasing concentration toward the interior, so that the silver atoms 2 are substantially detected only from the metallic material surface to a depth in the range of 5-30 µm, and the metallic silver, silver alloy or silver compound containing the silver atoms 2 is exposed on the surface 3. (In the present specification, "silver atoms" refers not only to "metallic silver" but also to the silver atoms of silver alloys and silver compounds.)

If the silver concentration in the metallic surface portion is greater than 10,000 ppm there will be a risk of change in the strength or corrosion resistance of the metallic material 1, while a concentration of less than 0.5 ppm will make it impossible to maintain a sufficiently useful level of antimicrobial properties for the metallic material surface as a whole.

If the silver atoms 2 serving as the antimicrobial component are distributed beyond a 30 µm depth from the surface, those silver atoms 2 will have little effect on the strength of weakness of the antimicrobial properties of the metallic surface. However, for metallic materials with large abrasion volumes, greater diffusion of silver into the interior will minimize loss of antimicrobial properties due to abrasion of the metallic surface.

The metals to be provided with antimicrobial properties are not particularly restricted, and examples include iron, stainless steel, aluminum alloys, etc., and these may be plated with nickel, chromium, tin, zinc, aluminum or the like, which are metals with weaker oligodynamic effects than silver.

Because the antimicrobial metallic material produced according to the process of the invention have excellent antimicrobial properties, they may be used as various types of metallic materials requiring high antimicrobial properties, including those used in medical instruments (scissors, tweezers, needles, scalpels, forceps, bowls, etc.), kitchen utensils (drainboards, colanders, sink triangular corners, etc.), tableware (cups, dishes, etc.), building material implements (handrails, doorknobs, bathtubs, metal fixtures, etc.), blades (kitchen knives, general use knives, cutters, saws, etc.), industrial materials (tanks, containers, etc.), handles, etc.

Since the metallic material 1 produced by the process of the invention which has been provided with antimicrobial properties in the manner described above has the silver atoms 2 diffused only in the surface portion of the metallic material, the amount of the silver, silver alloy or silver compound used may be economized, there is virtually no adverse effect on the properties of the main metallic material, and even if the surface 3 of the metallic material 1 undergoes some abrasion there is no loss of antimicrobial properties because the silver atoms 2 inside the surface portion will appear on the surface and exhibit their antimicrobial properties.

This metallic material 1 provided with antimicrobial properties maintains the antimicrobial properties of the silver atoms 2 and can exhibit those antimicrobial properties effectively by using less silver, which results in a lower cost.

### [Production process]

The production process (1) for an antimicrobial metallic material according to the invention comprises diffusing silver atoms from the metallic material surface into the interior of the metallic material surface portion.

The diffusion of the silver atoms may be accomplished, for example, by either of the following methods.
(1) A silver atom-containing coating layer including silver, a silver alloy or a silver compound is formed on the metallic material surface and subjected to heat treatment at a temperature of, for example, 100-1200°C at which the main metallic material is not adversely affected.
(2) A silver atom-containing coating layer including silver, a silver alloy or a silver compound is formed on the metallic material surface and subjected to heat treatment at a temperature of, for example, 100-1200°C at which the main metallic material is not adversely affected, while applying pressure.

Before the silver atom diffusion step, preferably the surface of the metallic material to be coated with the silver atom-containing coating layer is fully cleaned to remove contamination. Also, before the antimicrobial-treatment, the metallic material surface may be preprocessed by enameling, plating or the like.

The silver atom-containing coating layer including silver, a silver alloy or a silver compound may be formed on the metallic material surface by, for example, a method for coating of a coating solution, or by vapor deposition, CVD, ion plating, sputtering, flame spray coating, plating or the like.

The coating solution is prepared by dispersing or dissolving fine particles of the silver, silver alloy or silver compound in water or an organic solvent such as cellosolve, and mixing with a surfactant is desirable for improving the wettability of the metallic material surface. Examples of silver compounds include organic silver compounds such as silver citrate and silver lactate, and inorganic silver compounds such as silver chloride, silver sulfide, silver oxide and silver sulfate.

The concentration of the antimicrobial component, such as silver, in the coating solution is preferably 0.01-10 wt% in terms of silver atoms, because if the concentration is less than 0.01 wt% sufficient antimicrobial properties cannot be achieved, while if it is over 10 wt% a fusion film of the antimicrobial component will form on the metallic material surface, increasing the chances of residual contamination.

The particle size of the silver or other antimicrobial component in the coating solution as a colloid is no greater than 10 µm, and more preferably no greater than 0.1 µm, as this is most desirable from the standpoint of achieving superior diffusibility into the interior of the metallic material surface portion. There are no restrictions on the method of coating of the coating solution, and for example, brush coating, dipping, spraying and the like may be employed.

The method of heat treatment is not particularly restricted, and it may be appropriately selected from among resistance methods of resistance heating, countercurrent induction heating and electric heating of the metallic material itself. When the silver atom-containing coating layer is formed using CVD, sputtering, flame spray coating or a similar layering method in which the substrate (metallic material) is treated in a heated state, heat treatment at the aforementioned temperature of 100-1200°C is not always necessary.

The heat treatment atmosphere is not particularly restricted, but the use of a non-oxidizing atmosphere such as an argon atmosphere, for example, is preferred to avoid possible effects on the metallic material.

The heat treatment time is determined by the quality of the metallic material and the depth of diffusion of the silver atoms, but it is normally from a few minutes to about 100 hours. For example, in the case of stainless steel, about an hour is sufficient at a heat treatment temperature of 380°C, and 20-40 minutes is sufficient at 820°C.

The heat treatment step may be the same heat treatment step employed during the production process for the metallic material. For example, if the silver, silver alloy or silver compound is precoated on the metallic material surface prior to a heat treatment step by annealing, tempering, etc., this heat treatment step may serve as the heat treatment step for the antimicrobial metallic material.

The silver atoms may be diffused in the metallic material surface portion by pressure treatment in combination with the heat treatment.

The pressurization may be accomplished by roll pressurization, hydrostatic pressing, autoclave treatment or the like.

The production process (2) for the antimicrobial metallic material of the invention comprises ion implantation of silver atoms into the metallic material surface portion.

In ion implantation, the silver, silver alloy or silver compound is heated to vaporization for ionization of the silver atoms, and these are then accelerated to 10-100 eV and irradiated onto the metallic material surface for implantation into the interior.

The implantation rate of the silver ions may be about 10¹³-10¹⁷ ions/cm², which provides a silver atom concentration of 0.5-10,000 ppm in the metallic material surface portion.

For deeper diffusion of the silver atoms into the metallic material surface portion, it may be necessary for the ion implantation to be followed by heat treatment at a temperature which does not affect the main metal material. Also, it is preferred for the treatment surface of the metallic material to be adequately washed prior to implantation of the silver atoms, in order to remove any contamination. Before the antimicrobial-treatment, the surface of the metallic material may also be preprocessed by enabling, plating or the like.

In cases where a fusion film of the silver or other antimicrobial component is formed or impurities remain on the metallic material surface after heat treatment or ion implantation of the metallic material, the surface may be subjected to acid washing with a strong acid such as hydrochloric acid, nitric acid or sulfuric acid, or the metallic material surface may be polished to a depth in the range of 1-2 µm to remove the aforementioned fusion film or impurities.

Because the silver atoms are diffused within the metallic material surface portion, there is no loss of antimicrobial properties even if the uppermost section of the surface layer is removed, so that the original satisfactory antimicrobial properties may be exhibited.

### Examples

The following are concrete examples of metallic materials provided with antimicrobial properties.

### Example 1 Antimicrobial stainless steel scissors

A silver compound dispersion was prepared by adding a commercially available surfactant at 0.5% to a 1% aqueous solution of silver lactate. Stainless steel scissors were immersed in this dispersion for adhesion of the silver compound dispersion to 0.01 g/cm² in terms of silver atoms and, after drying at room temperature, the scissors were heat treated at a temperature of 400°C for 30 minutes and then acid washed with 5% hydrochloric acid to obtain antimicrobial stainless steel scissors.

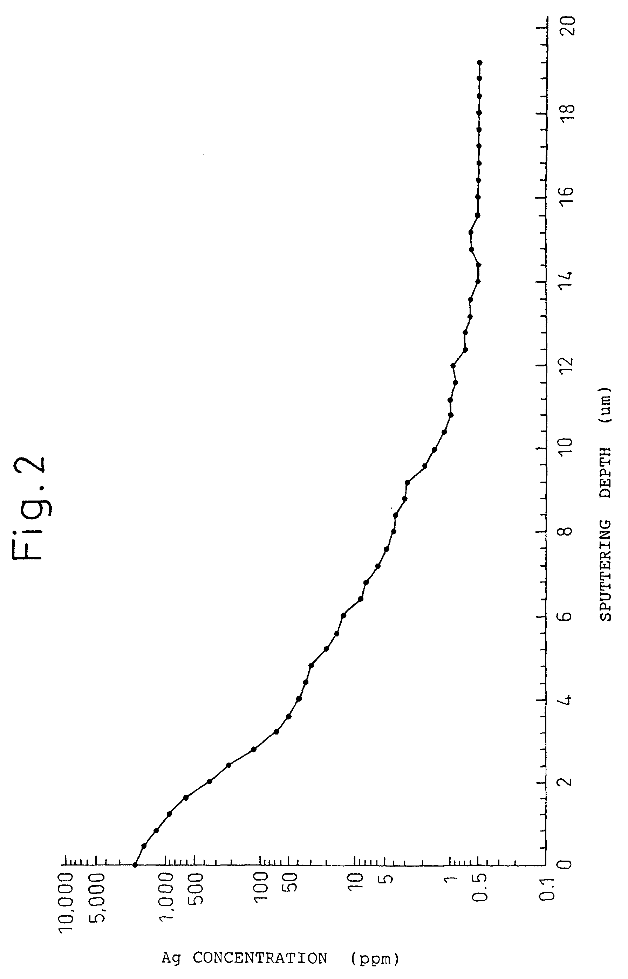

The state of diffusion of silver in the surface layer of the antimicrobial stainless steel scissors was analyzed by GDMS (glow discharge mass spectrometry) and a presence of silver atoms at 2000 ppm in the uppermost surface was confirmed, while the presence of silver atoms up to a depth of about 20 µm was also confirmed. Fig. 2 shows the state of silver atom distribution in the surface layer of the antimicrobial stainless steel scissors.

### Example 2 Antimicrobial steel blade

After forming a thin film of silver (0.01 µm thickness) on the surface of a steel blade by sputtering, the blade was heat treated at a temperature of 900°C for one minute, and then the uppermost surface of the blade was abraded to a depth of about 2 µm to obtain an antimicrobial steel blade.

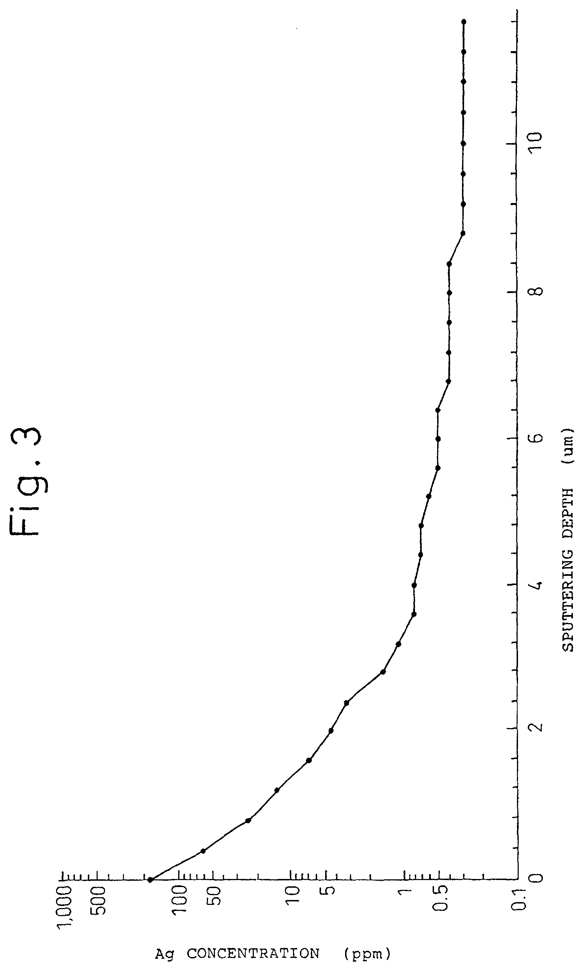

The state of diffusion of silver in the surface layer of the antimicrobial steel blade was analyzed by GDMS, and a presence of silver atoms at 200 ppm in the uppermost surface portion was confirmed, while the presence of silver atoms up to a depth of about 10 µm was also confirmed. Fig. 3 shows the state of silver atom distribution in the surface portion of the antimicrobial steel blade.

### Example 3 Antimicrobial nickel-chromium plated handle

Silver atoms were ion-implanted into the surface of a cast iron handle with a nickel-chromium plating on the surface, and this was acid washed with 5% hydrochloric acid to obtain an antimicrobial nickel-chromium plated handle.

The state of diffusion of silver in the surface portion of the antimicrobial nickel-chromium plated handle was analyzed by GDMS, and the presence of silver atoms at 50 ppm in the uppermost surface portion was confirmed, while the presence of silver atoms up to a depth of about 10 µm was also confirmed.

### Antimicrobial evaluation of metallic material products of Examples 1-3

The antimicrobial properties of the metallic materials obtained in Examples 1-3 were tested and evaluated by the film adhesion test established by the Silver and Other Inorganic Antimicrobial Agent Research Group.

The results are listed in Table 1.

The basic procedure for the film adhesion test was as follows. "Different microbial solutions containing Escherichia coli, Staphylococcus aureus, Bacillus subtilis, Bacillus pneumoniae, Salmonella and Pseudomonas, each preparation containing normal bouillon diluted to a concentration of 1/500 and having a bacterial concentration of about 10⁵ cfu/ml, were contacted with each the samples at an amount of 0.5 ml per 25 cm², and a polyethylene film of the same shape as the sample was placed on the bacterial solution.

After culturing the bacteria at a temperature of 35°C for 24 hours, the surviving cell count was measured by the agar plate method."

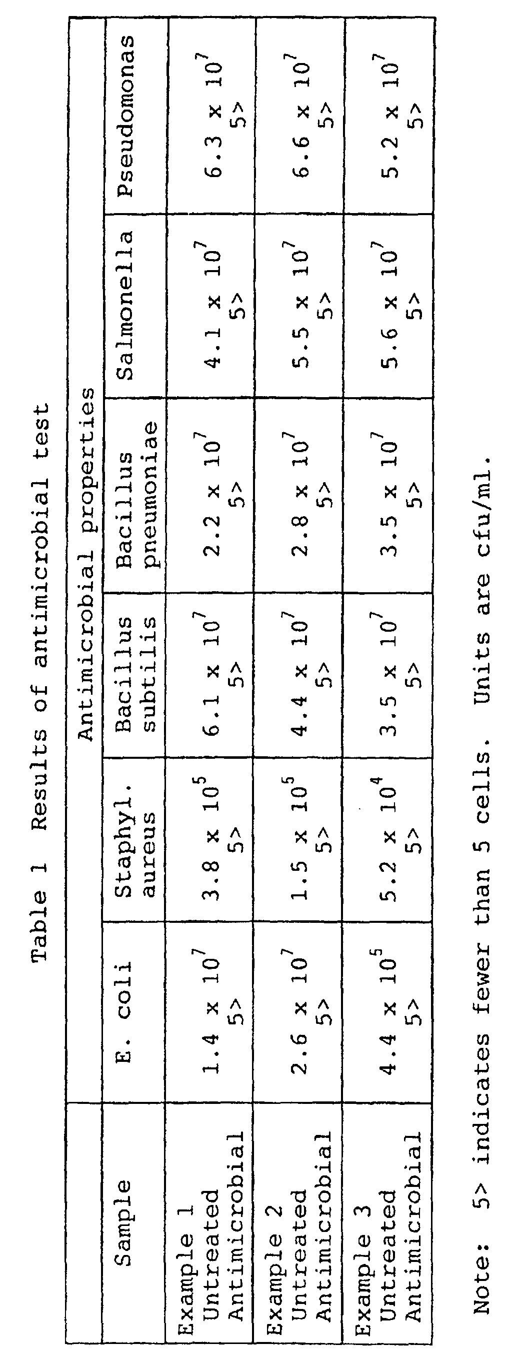

As clearly shown in Table 1, all of the bacterial species tested had fewer than 5 cells surviving on the antimicrobial metallic materials according to the invention, thus confirming a definite difference compared with the untreated products.

The antimicrobial metallic materials produced by the process of the invention can exhibit adequate antimicrobial properties by diffusion of silver atoms at a concentration of 0.5-10,000 ppm in the metallic material surface portion. The tools and utensils manufactured with such metallic materials can therefore provide satisfactory sanitary and hygienic levels required for such tools and utensils.

In addition, if the thickness of the silver atom-diffused layer in the metallic surface portion of the antimicrobial metallic material produced by the process of the invention is 30 µm or less, metallic materials can be provided with less silver usage but with the necessary antimicrobial properties, while the reduction in the amount of silver usage will allow cost savings.

Furthermore, by exposing a portion of the silver, silver alloy or silver compound in the metallic material surface portion of the antimicrobial metallic material of the invention through the metallic material surface, it is possible to exhibit effective antimicrobial properties without losing the properties of the main material, and the antimicrobial metallic material can be formed with a smaller amount of silver usage.

Moreover, according to production process (1) for an antimicrobial metallic material produced by the process of the invention, silver atoms are diffused from the metallic material surface into the interior of the metallic material surface portion, allowing production of a durable antimicrobial metallic material with a small amount of silver usage, while also lowering the cost of the antimicrobial metallic material.

Moreover, according to production process (2) for an antimicrobial metallic material of the invention, silver atoms are ion-implanted into the metallic material surface portion, so that effective antimicrobial properties can be provided with very low silver usage, thus effectively reducing production costs for the antimicrobial metallic material.

Furthermore, in both production processes (1) and (2) for antimicrobial metallic materials of the invention, successive removal of a portion of the surface portion will allow effective removal of fusion films and impurities which are formed on the surfaces of the antimicrobial metallic materials, without reducing their antimicrobial properties.

### Industrial Applicability

The production processes according to the invention make it possible to efficiently and economically provide metallic materials with excellent long-lasting antimicrobial properties.

## Claims

1. A process for producing an antimicrobial metallic material comprising heat-diffusing silver atoms from a surface into the inside of a metallic material to form a silver atom-diffused surface layer; and removing an uppermost surface portion of the silver atom-diffused surface layer to cause a resultant surface of the metallic material on which the silver atoms are distributed in a density of 0.5 to 10,000 ppm, to be exposed.

2. The process for producing an antimicrobial metallic material as claimed in claim 1 wherein, in the silver atom-diffused surface layer, the silver atoms are detected only from the metallic material surface to a depth in the range of 5 to 30 µm.

3. The process for producing an antimicrobial metallic material as claimed in claim 1 or 2 wherein, in the removing step, the upper most surface portion of the silver atom-diffused surface layer is polished to a depth of 1 to 2 µm, to remove a fusion film and impurities formed on the metallic material surface.

## Patentansprüche

1. Verfahren zur Herstellung eines mikrobiziden metallischen Materials, das die Diffusion von Silberatomen von der Oberfläche in ein metallisches Material in der Hitze, um eine von Silberatomen durchsetzte Oberflächenschicht zu bilden, und die Entfernung des äußeren Teils der mit Silberatomen durchsetzten Oberflächenschicht umfasst, um eine Oberfläche des metallischen Materials zu erzeugen, auf und in welcher die Silberatome mit einer Dichte von 0,5 bis 10 000 ppm verteilt sind.

2. Verfahren zur Herstellung eines mikrobiziden metallischen Materials nach Anspruch 1, wobei die Silberatome in der mit ihnen durchsetzten Oberflächenschicht ab der Oberfläche des metallischen Materials nur bis zu einer Tiefe von 5 bis 30 µm nachgewiesen werden.

3. Verfahren zur Herstellung eines mikrobiziden metallischen Materials nach Anspruch 1 oder 2, wobei in der Entfernungsstufe der äußere Teil der mit Silberatomen durchsetzten Oberflächenschicht bis zu einer Tiefe von 1 bis 2 um poliert wird, um einen Schmelzfilm und auf der Oberfläche des metallischen Materials gebildete Verunreinigungen zu entfernen.

## Revendications

1. Processus de production d'un matériau métallique antibactérien comprenant le fait de diffuser thermiquement des atomes d'argent à partir d'une surface à l'intérieur d'un matériau métallique pour former une couche de surface diffusée par des atomes d'argent ; et le fait de retirer une partie de surface la plus haute de la couche de surface diffusée par des atomes d'argent pour provoquer l'exposition d'une surface résultante du matériau métallique sur laquelle les atomes d'argent sont répartis à une densité de 0,5 à 10.000 ppm.

2. Processus de production d'un matériau métallique antibactérien selon la revendication 1, dans lequel, dans la couche en surface diffusée par des atomes d'argent, les atomes d'argent ne sont détectés de la surface du matériau métallique qu'à une profondeur allant de 5 à 30 µm.

3. Processus de production d'un matériau métallique antibactérien selon la revendication 1 ou 2, dans lequel, au cours de l'étape de retrait, la partie de surface la plus haute de la couche de surface diffusée par des atomes d'argent est polie jusqu'à une profondeur de 1 à 2 µm, pour retirer un film de fusion et des impuretés formées sur la surface du matériau métallique.
